(19) Europäisches Patentamt
European Patent Office
Office européen des brevets

(11) **EP 4 318 477 A1**

(12) **EUROPEAN PATENT APPLICATION**
published in accordance with Art. 153(4) EPC

(43) Date of publication:
**07.02.2024 Bulletin 2024/06**

(21) Application number: **21935332.3**

(22) Date of filing: **16.12.2021**

(51) International Patent Classification (IPC):
*G16B 5/00* (2019.01)          *G16B 30/10* (2019.01)
*G16B 40/20* (2019.01)          *G16B 20/20* (2019.01)
*G06N 3/08* (2023.01)          *G06N 3/04* (2023.01)
*G06N 20/00* (2019.01)          *C12Q 1/6869* (2018.01)

(52) Cooperative Patent Classification (CPC):
**C12Q 1/6869; G06N 3/04; G06N 3/08; G06N 20/00;
G16B 5/00; G16B 20/20; G16B 30/10; G16B 40/20**

(86) International application number:
**PCT/KR2021/019182**

(87) International publication number:
**WO 2022/211219 (06.10.2022 Gazette 2022/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.03.2021  KR 20210040869**

(71) Applicants:
• **Korea Advanced Institute of Science and
Technology
Daejeon 34141 (KR)**

• **Pentamedix Co., Ltd.
Sujeong-gu, Seongnam-si, Gyeonggi-do 13449
(KR)**

(72) Inventors:
• **CHOI, Jung Kyoon
Daejeon 34141 (KR)**
• **KIM, Jeong Yeon
Daejeon 34141 (KR)**
• **CHO, Dae Yeon
Seongnam-si, Gyeonggi-do 13540 (KR)**

(74) Representative: **Ostertag & Partner Patentanwälte
mbB
Azenbergstraße 35
70174 Stuttgart (DE)**

(54) **METHOD FOR PREDICTING T CELL ACTIVITY OF PEPTIDE-MHC, AND ANALYSIS DEVICE**

(57)    This method of predicting the T cell activation of peptide-MHC comprises the steps in which an analysis apparatus: receives genetic data of a patient; identifies, on the basis of the genetic data, a first amino acid sequence of a major histocompatibility complex (MHC) and a second amino acid sequence of antigen generated by tumor cells; produces a matrix indicating the interrelationship between the first amino acid sequence and the second amino acid sequence in a single amino acid unit; and inputs the matrix to a trained neural network model to determine whether the T cells secrete at least a threshold amount of cytokine as a result of the binding of the MHC and the antigen.

FIG. 1

**Description**

[Technical Field]

The following description relates to a technique for predicting T cell activation for antigen peptide-major histocompatibility complex (MHC).

[Background Art]

A neoantigen is a tumor cell-specific protein. The neoantigen is expressed due to tumor cell-specific mutation. Epitopes of the neoantigen are expressed on major histocompatibility complexes (MHC) located on surfaces of tumor cells, and T cells recognize MHC-epitopes to trigger immune responses.
Cancer immunotherapy is therapy that activates a body's immune system to kill tumor cells. Research is being conducted to discover effective neoantigens in the field of cancer immunotherapy.

[Disclosure]

[Technical Problem]

The following description provides an in silico technique for discovering a neoantigen having high reactivity with a T cell.

[Technical Solution]

In one general aspect, there is a method of predicting the T cell activation for peptide-major histocompatibility complex (MHC) includes: receiving, by an analysis apparatus, genetic data of a patient; identifying, by the analysis apparatus, a first amino acid sequence of MHC and a second amino acid sequence of antigen generated by a tumor cell on the basis of the genetic data; producing, by the analysis apparatus, a matrix indicating an interrelationship between the first amino acid sequence and the second amino acid sequence in a single amino acid unit; and inputting, by the analysis apparatus, the matrix to a trained neural network model to determine whether the T cell secretes cytokine greater than or equal to a threshold value according to a binding of the MHC and the antigen.
In another aspect, there is an analysis apparatus for predicting T cell activation for peptide-MHC includes: an input device configured to receive genetic data of a patient; a storage device configured to store a neural network model that predicts a cytokine secretion amount of a T cell based on a matrix representing an interrelationship of an amino acid sequence of MHC and an amino acid sequence of antigen generated by a tumor cell; and an arithmetic device configured to identify a first amino acid sequence of the MHC and a second amino acid sequence of the antigen generated by the tumor cell from the genetic data, produce a matrix representing the interrelationship between the first amino acid sequence and the second amino acid sequence in a single amino acid unit, and input the produced matrix to the neural network model to determine whether the MHC-antigen of the patient induces interferon-γ secretion of the T cell.

[Advantageous Effects]

The technologies described below use a deep learning model to quickly select neoantigens with high T cell activation among candidate peptides of a patient. The technologies described below predict T cell activation for antigen peptide-major histocompatibility complex (MHC) with high accuracy using an interferon-γ secretion amount as a reference.

[Brief description of Drawings]

FIG. 1 is an example of a system for predicting T cell activation for peptide-major histocompatibility complex (MHC).
FIG. 2 is an example of a process of developing a personalized anticancer vaccine.
FIG. 3 is an example of a process of training a neural network model.
FIG. 4 is an example of a process of producing a matrix illustrating an interaction of peptide-MHC.
FIG. 5 is an example of a process of predicting T cell activation for peptide-MHC.
FIG. 6 is an example of an analysis apparatus for predicting T cell activation for peptide-MHC.
FIG. 7 is an example of experimental results verifying a neural network model.
FIG. 8 is another example of experimental results verifying a neural network model.

[Exemplary embodiments of invention]

The present disclosure may be variously modified and have several exemplary embodiments. Therefore, specific exemplary embodiments of the present disclosure will be illustrated in the accompanying drawings and be described in detail. However, it is to be understood that the present invention is not limited to the specific exemplary embodiments, but includes all modifications, equivalents, and substitutions without departing from the scope and spirit of the present invention.

Terms such as "first," "second," "A," "B," and the like may be used to describe various components, but the components are not to be interpreted to be limited to the terms and are used only for distinguishing one component from other components. For example, a first component may be named a second component and the second component may also be similarly named the first component, without departing from the scope of the present disclosure. The term "and/or" includes a combination of a plurality of related described items or any one of the plurality of related described items.

It should be understood that the singular expression include the plural expression unless the context clearly indicates otherwise, and it will be further understood that the terms "comprises" and "have" used in this specification specify the presence of stated features, steps, operations, components, parts, or a combination thereof, but do not preclude the presence or addition of one or more other features, numerals, steps, operations, components, parts, or a combination thereof.

Prior to the detailed description of the drawings, it is intended to clarify that the components in this specification is only distinguished by the main functions of each component. That is, two or more components to be described below may be combined into one component, or one component may be divided into two or more for each subdivided function. In addition, each of the constituent parts to be described below may additionally perform some or all of the functions of other constituent parts in addition to the main functions of the constituent parts, and some of the main functions of the constituent parts may be performed exclusively by other components.

In addition, in performing the method or the operation method, each of the processes constituting the method may occur differently from the specified order unless a specific order is explicitly described in context. That is, the respective steps may be performed in the same sequence as the described sequence, be performed at substantially the same time, or be performed in a sequence opposite to the described sequence.

Terms used in the following description will be described.

An antigen is a substance that induces an immune response.

A neoantigen is a tumor cell-specific antigen resulting from mutation or post-translational modification of tumor cells. The neoantigen may include a polypeptide sequence or a nucleotide sequence. Here, a mutation may include any genomic or expression alteration that causes a frameshift, insertion, deletion, substitution, splice site alteration, genomic rearrangement, gene fusion, or de novo open reading frame (ORF). In addition, a mutation may also include a splice variant. Post-translational modifications specific to tumor cells may include aberrant phosphorylation. The post-translational modifications specific to tumor cells may also include proteasome-generated spliced antigens.

Epitope may be referred to as a specific part of antigen to which an antibody or a T-cell receptor usually binds.

Major histocompatibility complex (MHC) is a peptide structure that acts as a mediator to recognize a target substance of an immune response as an antigen. Human MHC is called a human leukocyte antigen (HLA). Hereinafter, the MHC is used as a meaning including the human HLA.

Peptide is an amino acid polymer. Technologies described below correspond to a technique for discovering a neoantigen. The peptide used in the following description is an amino acid polymer or an amino acid sequence expressed in a tumor cell. Accordingly, the following peptide may be a tumor-specific amino acid polymer or amino acid sequence expressed on a surface of a tumor cell.

Peptide-MHC (pMHC) or a peptide-MHC complex is a structure of peptide and MHC expressed on a surface of a tumor cell. The T cell recognizes the peptide-MHC complex and performs the immune response.

A binding degree is a degree of binding between the MHC and the peptide. Binding preference or binding affinity is a degree of binding affinity between an MHC molecule and the peptide.

A sample is a single cell or multiple cells, cell fragments, a body fluid, etc., in a subject to be analyzed.

A subject includes a cell, a tissue, or an organism. Generally, the subject is obtained from a patient with a specific tumor. The subject is basically a human, but is not limited thereto.

An exome is a subset of a genome that encodes protein. The exome may refer to a set of exons present in a cell, a cell group, or an organism.

Genetic data refers to genetic information calculated by analyzing a sample. For example, genetic data may include a base sequence obtained from deoxyribonucleic acid (DNA), ribonucleic acid (RNA), or protein from cells, tissues, etc., gene expression data, genetic variation with standard genetic data, DNA methylation, etc. The genetic data may be obtained through a traditional sequencing method, next-generation sequencing (NGS), or the like. Genetic data is generally digital data and may be calculated in the form of a file of a specific format (e.g., FATSQ).

Machine learning is a field of artificial intelligence, and refers to a field of algorithms developed so that a computer may be learned. The learning model includes a decision tree, a random forest (RF), a K-nearest neighbor (KNN), a naive Bayes, a support vector machine (SVM), an artificial neural network, or the like. Technologies described below may use an artificial neural network. The following description will focus on an artificial neural network or a neural network model.

The artificial neural network is a statistical learning algorithm that mimics a biological neural network. Various neural network models are being studied. Recently, a deep learning network (DNN) is attracting attention. The DNN is an artificial neural network model composed of several hidden layers between an input layer and an output layer. Similar to general artificial neural networks, the DNN may model complex non-linear relationship. Various types of DNN models have been studied. Examples of the DNNs include a convolutional neural network (CNN), a recurrent neural network (RNN), a restricted Boltzmann machine (RBM), a deep belief network (DBN), a generative adversarial network (GAN), relation networks (RLs), and the like.

An analysis apparatus is a device that discovers a neoantigen of a specific tumor from a patient's sample. The analysis apparatus predicts T cell activation for specific peptide-MHC. The analysis apparatus may process and analyze data using an installed program or code.

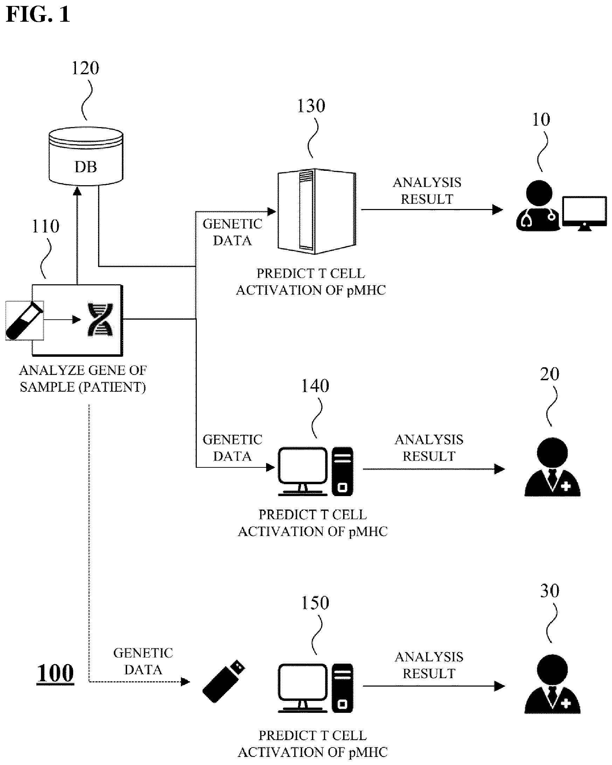

FIG. 1 is an example of a system 100 for predicting T cell activation for peptide-MHC. In FIG. 1, analysis apparatus 130, 140, and 150 predict T cell activation. In FIG. 1, the analysis apparatus is illustrated in the form of a server 130 and computer terminals 140 and 150. Meanwhile, the analysis apparatus 130, 140, and 150 may be implemented in various forms.

The gene analysis apparatus 110 generates genetic data by analyzing a patient's sample. For example, the gene analysis apparatus 110 may be an NGS analysis apparatus. Since a peptide expressed by a tumor cell is an analysis target, the gene analysis apparatus 110 may perform sequencing targeting an exome. A detailed description of whole exome sequencing is omitted. The gene analysis apparatus 110 may store the generated genetic data in a separate DB 120.

The server 130 receives genetic data from the gene analysis apparatus 110 or DB 120. The server 130 provides a service for predicting T cell activation by analyzing genetic data.

The computer terminal 140 receives genetic data from the gene analysis apparatus 110 or the DB 120. The computer terminal 140 predicts T cell activation by analyzing genetic data.

The computer terminal 150 receives genetic data through a medium (e.g., a Universal Serial Bus (USB), a secure digital (SD) card, etc.) in which the genetic data generated by the gene analysis apparatus 110 is stored. The computer terminal 150 predicts T cell activation by analyzing genetic data.

The analysis apparatus 130, 140, and 150 predict the degree of T cell activation for peptide-MHC, which is the current analysis target, and may discover the current peptide as a neoantigen candidate when the T cell activation is greater than or equal to a threshold value. The analysis apparatus 130, 140, and 150 may input information on the peptide-MHC to a previously constructed neural network model to predict the degree of T cell activation. A process of predicting, by the analysis apparatus 130, 140, and 150, T cell activation for specific peptide-MHC using a neural network model will be described below.

Users 10, 20, and 30 may be researchers or medical staffs developing neoantigens and vaccines. The users 10, 20, and 30 may confirm the degree of T cell activation for specific peptide-MHC in the sample. In addition, the users 10, 20, and 30 may identify valid neoantigens for the sample. The user 10 may access the server 130 through a user terminal (PC, smart phone, etc.) and confirm the analysis result performed by the server 130. The user 20 may confirm the analysis result through the computer terminal 140 used by the user 20. The user 30 may confirm the analysis result through the computer terminal 150 used by the user 30.

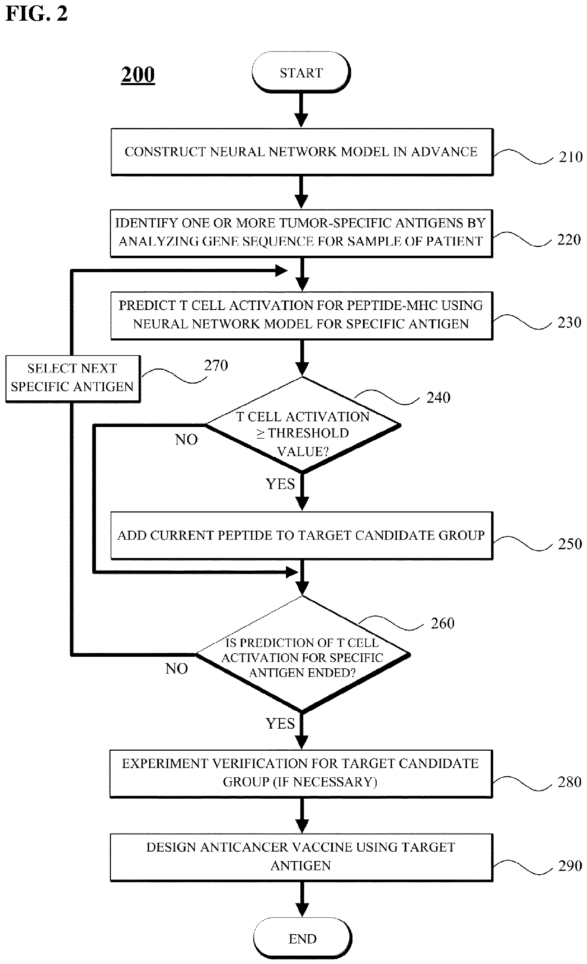

FIG. 2 is an example of a process of developing a personalized anticancer vaccine (200). FIG. 2 includes a process of predicting, by the analysis apparatus, T cell activation using a neural network model and discovering a neoantigen according to the prediction result.

The analysis apparatus constructs a neural network model by training a neural network model with training data (210). The process of training the neural network model will be described below. The process of training the neural network model may be performed by a separate computer device other than the analysis apparatus. That is, a subject training the neural network model and a subject analyzing using the neural network model may be different from each other.

The analysis apparatus receives a gene sequence analysis result (genetic data) of a patient's sample (e.g., tumor tissue). The analysis apparatus may identify tumor-specific mutation sequences in genetic data. The analysis apparatus may identify mutation sequences in tumor tissue sequences based on normal tissue sequences or reference sequences. The analysis apparatus may identify a tumor-specific mutation sequence as a tumor-specific antigen (220). That is, the analysis apparatus may select the tumor-specific mutation sequence as a neoantigen candidate. The analysis apparatus may select multiple neoantigen candidates. It is assumed and described that the multiple neoantigen candidates have been identified.

The analysis apparatus predicts T cell activation by selecting a specific candidate from among the neoantigen candidates. The analysis apparatus may identify a gene sequence for a specific candidate using genetic data. The analysis apparatus may determine an amino acid sequence of a candidate antigen based on the gene sequence for the specific candidate. In addition, the analysis apparatus may identify the MHC sequence of the patient using genetic data. The analysis apparatus may determine an amino acid sequence of MHC based on the MHC sequence.

The analysis apparatus predicts T cell activation using a previously constructed neural network model for candidate antigens (230). The analysis apparatus predicts T cell activation by inputting information on candidate antigens to a neural network model. The analysis apparatus may predict the T cell activation by inputting the amino acid sequence of the candidate antigen and the amino acid sequence of MHC to the neural network model. The analysis apparatus may produce a matrix representing the interaction or affinity between the candidate antigen and the MHC based on the amino acid sequence of the candidate antigen and the amino acid sequence of MHC. The analysis apparatus can predict T cell activation for candidate antigen by inputting the generated matrix to a neural network model. The operation of the neural network model will be described below.

The neural network model outputs information on whether the input candidate antigen is active in T cells. For example, the neural network model may output whether T cells are active or inactive for a candidate antigen to be analyzed. Whether the T cells are active may be determined by whether activity is greater than or equal to a certain threshold value.

The analysis apparatus determines whether the T cell activation is greater than or equal to a threshold value (240). Meanwhile, it may be determined whether the T cells are active based on the cytokine amount secreted by the T cells. The analysis apparatus adds the current candidate antigen (peptide) to the target candidate group when the T cell activation for current candidate antigen is greater than or equal to the threshold value (YES in 240) (250). The target candidate group is composed of tumor-specific neoantigen candidates that may be targeted for immune cancer therapy.

The analysis apparatus may confirm whether the prediction of T cell activation for specific antigen of the sample has been completed (260). When the prediction of the T cell activation for candidate antigen of the sample is not completed (NO in 260), the analysis apparatus repeats a process of selecting a next specific antigen, that does not predict T cell activation, from among candidate antigens (270) and determining the T cell activation for the corresponding antigen. The analysis apparatus even performs the process of extracting the target candidate groups.

When the prediction of the T cell activation for all candidate antigens is completed, researchers may perform an additional validation experiment for the current target candidate group (280). Furthermore, researchers may design vaccines targeting neoantigens with high T cell activation for current patients (290). The anticancer vaccine produced through this process is tailored to the patient and targets only tumor cells.

The process of constructing the above-described neural network model will be described. Researchers describe the process of constructing an actual neural network model.

Researchers collected information on peptide-MHC (pMHC) from open DBs. The open DB may include IEDB and the like. Researchers collected pMHC data for human and mouse from IEDB, IMMA2, MHCBN, and separate sources. In addition, researchers collected information on the T cell activation in association with the pMHC. The T cell activation was determined based on the cytokine amount secreted by T cells. More specifically, researchers evaluated the T cell activation for specific pMHC based on the interferon gamma (IFNγ) secretion amount secreted by T cells. To this end, researchers selected data that has the IFNγ secretion amount of T cells for the corresponding pMHC among the pMHC data in the open DB.

The data collected by the researchers includes an HLA type and a peptide length. The peptide length is 9mer for MHC class I and 15mer for MHC class II. In addition, immunogenicity label values for each pMHC were also used.

Unlike the MHC I, in the MHC II, HLA-DP and HLA-DQ are heterodimers, so experimental data are given as HLA-DQA/HLA-DQB pairs. As will be described below, since a molecular distance between the antigen and the MHC was used as training data, researchers used only a beta chain directly acting on the antigen. The training data was adjusted to balance antigenic and non-antigenic data. Finally, researchers prepared 13,128 MHC I data and 6,650 MHC II data. The data collected by the researchers are shown in Table 1 below. In Table 1, "individual research" refers to data obtained through individual research. Immunogenic peptides refer to tumor-specific neoantigens.

[Table 1]

| Type | Source | Total Peptides | Immunogenic Peptides | Peptide Length |
|---|---|---|---|---|
| Human - MHC class I | IEDB | 15925 | 4045 | 9 |
| Human - MHC class I | individual research | 2613 | 557 | 8~11 |
| Human - MHC class I | IMMA2 | 1085 | 558 | 9 |
| Mouse - MHC class I | IEDB | 4373 | 1162 | 9 |
| Mouse - MHC class I | MHCBN | 324 | 260 | 9 |

(continued)

| Type | Source | Total Peptides | Immunogenic Peptides | Peptide Length |
|---|---|---|---|---|
| Mouse - MHC class I | individual research | 258 | 165 | 9~15 |
| Human - MHC class II | IEDB | 6841 | 3968 | 15 |
| Human - MHC class II | MHCBN | 1014 | 416 | 15 |
| Mouse - MHC class II | IEDB | 3461 | 452 | 15 |
| Mouse - MHC class II | MHCBN | 128 | 125 | 15 |

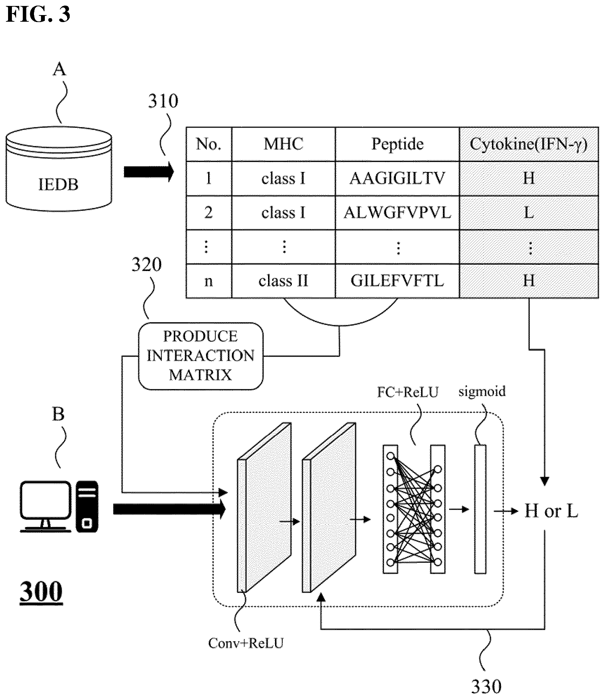

FIG. 3 is an example of a process of training a neural network model (300).

A peptide DB A may store information related to peptide-MHC. FIG. 3 illustrates only an open DB such as IEBD. However, the training data may include an open DB and data obtained by a researcher (developer) through a separate experiment. The peptide DB A may be a device existing on a network. Alternatively, the peptide DB A may be a device connected to or embedded in the computer device B.

The computer device B may learn the neural network model using the training data. The computer device B may be an analysis apparatus for predicting T cell activation or may be a dedicated device for training.

The computer device B extracts training data from the peptide DB A (310). As illustrated in FIG. 3, the training data may include an MHC class, an amino acid sequence of antigen candidate, and the IFNγ secretion amount of T cells for the corresponding peptide-MHC. The IFNγ secretion amount may be divided into a case (high, H) where it is greater than or equal to the threshold value and a case (low, L) where it is less than the threshold value, which is a reference for determining the T cell activation.

The computer device B produces a matrix representing the interrelationship between the peptide and the MHC for the peptide-MHC pair for the specific antigen to be analyzed (320). The interrelationship may refer to affinity between the peptide and the MHC. A process of producing a matrix will be described below.

The computer device B predicts the T cell activation for current peptide-MHC by inputting the produced matrix to the neural network model. The neural network model outputs information of the T cell activation (IFNγ secretion amount: high) or inactivity (IFNγ secretion amount: low) of peptide-MHC. The computer device B updates a weight of the neural network model based on the currently input label value (IFNγ secretion amount) for the peptide-MHC (330). The neural network model may be learned through a backpropagation process.

Researchers predicted the T cell activation for peptide-MHC using the CNN model. Of course, it may be possible to predict the T cell activation using the neural network model other than the CNN. The neural network model will be described, focusing on the CNN.

The CNN may include a convolution layer (Conv), a pooling layer, and a fully connected layer (FC). A plurality of convolution layers and pooling layers may be repeatedly arranged.

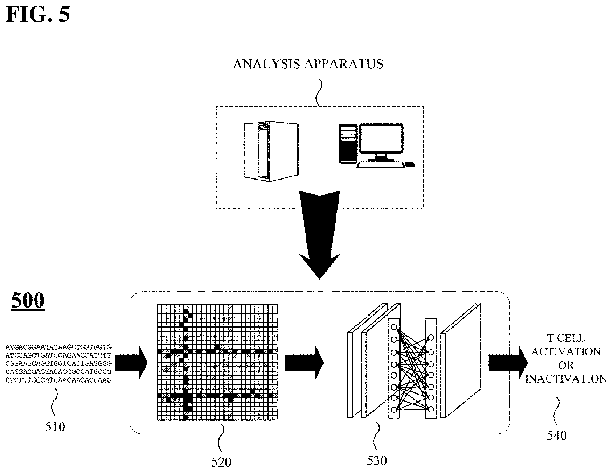

The CNN model 400 predicts a peptide-MHC binding degree based on input data (interaction map). The CNN model 400 includes a plurality of convolution layers 410 and 420, a fully connected layer 430, and an output layer 440. As illustrated in FIG. 5, the convolution layer may be composed of two layers.

The convolution layer performs a convolution operation on the input data and outputs a value obtained by applying a rectified linear unit (ReLU) function to the convoluted value. The convolution operation is an operation that multiplies a weight matrix for the input value. The weight may be updated through the training process. The convolution layer extracts interaction features for the peptide-MHC. Meanwhile, the input data may include parameters representing the degree of interaction of amino acid pairs.

The fully connected layer integrates the input information. The fully connected layer receives the value output from the convolution layer as an input. The fully connected layer may perform the ReLU operation.

The output layer uses a sigmoid function to output information on the degree of the given T cell activation for peptide-MHC or whether the T cell is active.

A value finally output by the neural network model may be a value between 0 and 1. The analysis apparatus may determine the activity or inactivity of the T cells by comparing the value output by the neural network model with the threshold value.

Further explanation will be given based on the model illustrated in FIG. 3.

The convolution layer performs convolution using the specific number of kernels or weight matrices. The convolution may be a one-dimensional or two-dimensional operation, etc. All the convolution results are transformed by the ReLU. The ReLU transforms negative values into zero. FIG. 3 illustrates two convolutional layers.

A first convolutional layer detects combination patterns in the input data. The first convolution layer may use a window with a moving distance of 1. The operation of the convolution layer is shown in Equation 1 below. A second convolution layer may have the same structure as the first convolution layer. Alternatively, the second convolution layer may have a window size or a stride width different from that of the first convolution layer.

[Equation 1]

$$\text{convolution}(X)_{ik} = \text{ReLU}\left(\sum_{m=0}^{M-1}\sum_{n=0}^{N-1} W_{mn}^k X_{i+m,n}\right)$$

X denotes the input data, i denotes an index indicating a location of an output, and k indicates an index of a kernel. Each convolution kernel $W_k$ corresponds to a weight matrix of size M×N. M denotes a window size, and N denotes the number of input channels.

The pooling layer may not be used. The pooling is a process of reducing a dimension of data. Even amino acids that are relatively far from each other may affect the interaction of the peptide-MHC complex with the T cell receptor. Therefore, the CNN may extract features while maintaining the size of input data without using the pooling layer.

The fully connected layer FC takes all outputs from the second convolutional layer as an input. The fully connected layer integrates the input values output from the previous layer. The fully connected layer performs the ReLU(WX) function. X denotes an input value, and W denotes a weight matrix for the fully connected layer.

The output layer may output a value between 0 and 1 according to the sigmoid function. The value output from the output layer is the activity H or inactivity L of the T cell. The output layer performs the sigmoid function Sigmoid(WX). X denotes the input value, and W denotes the weight matrix for the sigmoid output layer. Meanwhile, the output layer may use an activation function such as softmax or ReLU other than the sigmoid.

The CNN model is trained in a direction of minimizing an objective function. The training process corresponds to a process of optimizing weights used in the CNN model. For example, the weight optimization may use a gradient descent method.

The objective function is defined as a sum of negative log likelihood (NLL) and regularization term. The objective function for the CNN model may be expressed as Equation 2 below.

[Equation 2]

$$\text{Objective} = \text{NLL} + \lambda_1 \|W\|_2^2 + \lambda_2 \|H^{-1}\|_1$$

$$\text{NLL} = -\sum_s \sum_t \log(Y_t^s f_t(X^s) + (1 - Y_t^s)(1 - f_t(X^s)))$$

s denotes an index of the training data. t denotes an index of the interaction feature. $Y_t^s$ denotes a label value (0 or 1) for T cell activation of the training data s. $f_t(X^s)$ denotes the result predicted by the neural network model for the T cell activation of the input data $X^s$.

Meanwhile, MHC I and MHC II have different functional features and different protein lengths. Therefore, it is desirable to prepare neural network models for the MHC I and the MHC II, respectively. Researchers also construct a neural network model separately by individually using the training data for the MHC I and the MHC II.

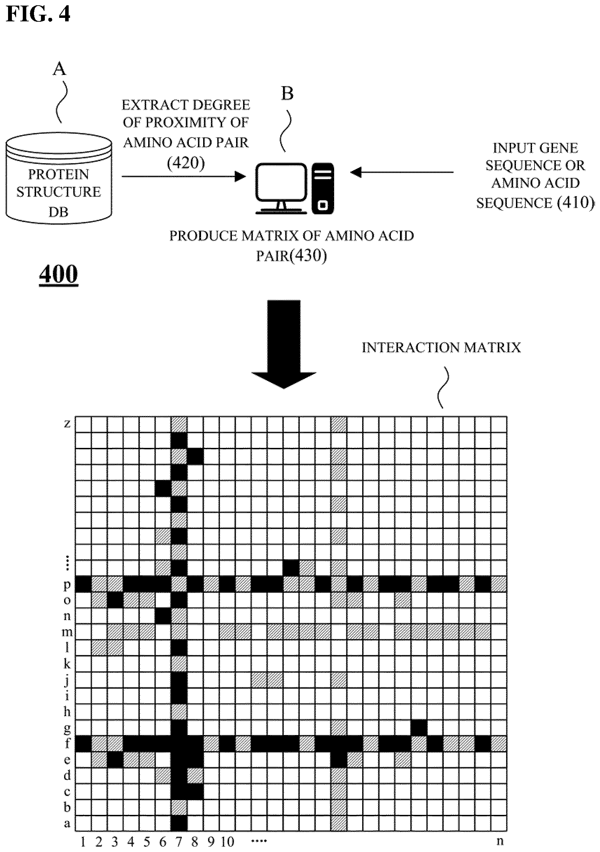

The neural network model receives a matrix for the peptide-MHC. The computer device produces the matrix for the peptide-MHC in the training process. The analysis apparatus produces matrices for each peptide-MHC in the analysis process. FIG. 4 is an example of a process of producing a matrix representing the interaction of the peptide-MHC (400). For convenience of description, it is assumed in FIG. 4 that the computer device B produces the matrix. Meanwhile, the computer device may be a PC, a server, or the like.

The computer device B receives amino acid sequences for peptide-MHC (410). The computer device may receive amino acid sequences through an input device, a storage medium, or communication. The amino acid sequences are an amino acid sequence of MHC and an amino acid sequence of antigen. Alternatively, the computer device may store a specific MHC amino acid sequence in advance and receive only the amino acid sequence of the antigen.

The computer device B produces a matrix of a pair of the amino acid sequence of MHC and the amino acid sequences of antigen. In the amino acid sequence of MHC, individual amino acids may be identified as 1 to n in sequence. In addition, in the amino acid sequence of the antigen, individual amino acids may be identified as a to z in sequence.

The computer device B determines interaction values for each pair of amino acids for the amino acid sequence (named first amino acid sequence) of MHC and the amino acid sequence (named second amino acid sequence) of the antigen. For example, the computer device determines interaction values for amino acid 1 of the first amino acid sequence and amino acid a of the second amino acid sequence. In this way, the computer device determines the interaction values for all the amino acid pairs that may be composed of the first amino acid sequence and the second amino acid sequence.

The computer device B may determine an interaction value on a specific amino acid by referring to the previously known structure of protein. The protein structure DB A stores information on structures of previously known proteins. The protein structure DB A may hold information on amino acids constituting the protein structure and a distance between the amino acids. The protein structure DB A may hold information on a plurality of protein structures.

The computer device B may determine a distance (proximity) between a specific first amino acid of the first amino acid sequence and a specific second amino acid of the second amino acid sequence by referring to the protein structure DB A. The protein structure DB A may hold distance information on a plurality of identical amino acid pairs. The computer device B may determine the interaction value of the first amino acid-second amino acid pair based on various criteria. For example, (i) the computer device B may determine an average distance of the first amino acid-second amino acid pair in the protein structure DB A as the interaction value of the first amino acid-second amino acid pair. (ii) The computer device B may determine the interaction value of the first amino acid-second amino acid pair based on the proximity frequency of the first amino acid-second amino acid in the protein structure DB A. The computer device B may determine that the corresponding amino acid pair is close when the first amino acid-the second amino acid in the protein structure DB A is located within a predetermined reference distance in a secondary space or a tertiary space. Now, (ii) the computer device B may determine the interaction value of the first amino acid-second amino acid pair based on the proximity frequency of the first amino acid-second amino acid in the protein structure DB A. The computer device B may determine, as the interaction value, the number of times the first amino acid and the second amino acid are in close proximity in the protein structure DB A. Alternatively, the computer device B may determine the interaction value by processing the frequency of proximity between the first amino acid and the second amino acid in the protein structure DB A.

The interaction values between the amino acid pairs may be determined in units of regions where the protein structure is constantly divided. The interaction value between the amino acids may be determined based on the distance between $C\alpha$ (alpha carbon) atoms existing in the protein structure.

The computer device B extracts proximity information (distance or proximity frequency, etc.) of a specific amino acid pair by referring to the protein structure DB A (420). The computer device B produces a matrix by determining the interaction values for each amino acid pair constituting the first amino acid sequence and the second amino acid sequence (430). The matrix represents the interaction of the amino acid sequences, and may also be named the interaction matrix. The matrices for the first amino acid sequence and the second amino acid sequence are composed of information representing the degree of interaction (affinity or proximity) for each amino acid pair.

An example of the interaction map is illustrated at the bottom of FIG. 4. The interaction map is a two-dimensional matrix with a horizontal axis and a vertical axis. The horizontal axis corresponds to amino acid sequences of MHC labeled as 1 to n, and the vertical axis corresponds to amino acid sequences of antigen labeled as a to z.

The matrix includes the interaction values for each pair of amino acids. The interaction value may be a numerical value. Furthermore, the matrix may be in the form of a map representing the degree of interaction with a constant color.

Meanwhile, the amino acid sequence length of antigen may be different depending on source data or MHC class. Accordingly, the computing device may pad the matrix based on the largest input data.

FIG. 5 is an example of a process 500 for predicting T cell activation for peptide-MHC.

The analysis apparatus receives genetic data of a sample (510). The sample may be tissue from a patient with a specific tumor. The genetic data may include information on a plurality of antigens. For convenience of description, it will be described based on one peptide-MHC.

Meanwhile, the analysis apparatus may select a previously construct neural network model according to the MHC class. As described above, different neural network models may be prepared according to the MHC class. Accordingly, the analysis apparatus may select a matching neural network model according to the MHC class of the current analysis target, and then proceed with the analysis process.

The analysis apparatus extracts an amino acid sequence of MHC and an amino acid sequence of antigen from genetic data. The analysis apparatus may use a program or model that predicts the MHC structure. For example, the analysis apparatus may predict an HLA structure using HLAminer. In addition, the analysis apparatus may identify the amino acid sequence of the antigen from the genetic data using a certain program. For example, the analysis apparatus may detect an amino acid sequence of antigen by searching for flanking amino acid sequences of nonsynonymous mutations from genetic data using an idfetch program.

As described in FIG. 4, the analysis apparatus can generate a matrix for the amino acid sequence of MHC and the amino acid sequence of the antigen (520).

The analysis apparatus performs analysis by inputting the produced matrix to the neural network model (530). The analysis apparatus may determine whether the current analysis target, the T cell activation for peptide-MHC, is determined based on information (T cell activation or inactivity) output on the matrix input by the neural network model (540).

The analysis apparatus may determine whether the T cells are active by comparing the value output by the neural network model with the threshold value. Researchers constructed individual neural network models for the MHC I and the MHC II. In the case of the neural network model constructed using the training data described in Table 1, the neural model of MHC I neural network model outputs a value greater than 0.5 to determine the T cell activation, and the neural network of MHC II outputs a value greater than 0.7 to determine the T cell activation.

Furthermore, the analysis apparatus may determine the antigen to be a target candidate for an anticancer vaccine when the T cell of peptide-MHC is active.

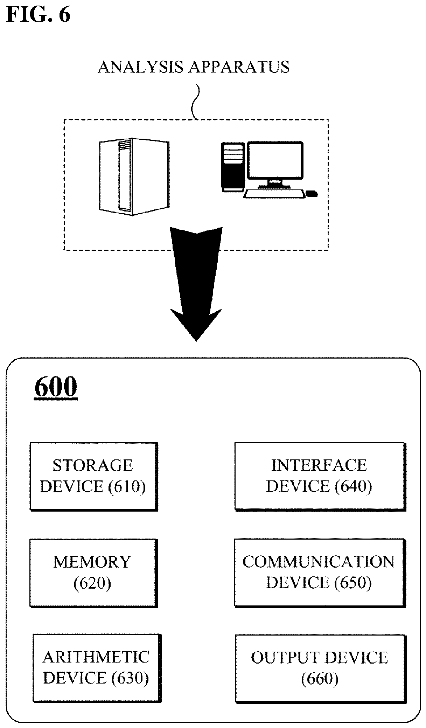

FIG. 6 is an example of an analysis apparatus 600 for predicting T cell activation for peptide-MHC. The analysis apparatus 600 is a device corresponding to the analysis apparatus 130, 140, or 150 of FIG. 1.

The analysis apparatus 600 may predict the peptide-MHC binding degree using the neural network model described above. The analysis apparatus 600 may be physically implemented in various forms. For example, the analysis apparatus 600 may have the form of a computer device such as a PC, a smart device, a server of a network, and a data processing-only chipset.

The analysis apparatus 600 may include a storage device 610, a memory 620, an arithmetic device 630, an interface device 640, a communication device 650, and an output device 660.

The storage device 610 stores a neural network model predicting the degree of T cell activation. The neural network model is as described above. The neural network model should be trained in advance. The neural network model can output a cytokine secretion amount corresponding to a measure of T cell activation. For example, the neural network model may output the amount of IFNγ secreted by T cell. The neural network model may output information representing T cell activation (secretion of a large amount of IFNγ) or inactivity (secretion of a small amount of IFNγ or no secretion of IFNy).

Furthermore, the storage device 610 may store a program, a source code, or the like required for data processing.

The storage device 610 may store the input genetic data. The storage device 610 may store an amino acid sequence of antigen to be analyzed. The storage device 610 may store an amino acid sequence of MHC to be analyzed.

The storage device 610 may store a program for identifying the sequences of MHC and/or antigen from genetic data.

The storage device 610 may store the degree of T cell activation for specific peptide-MHC, which is an analysis result. The storage device 610 may store the above-described neoantigen candidates.

The memory 620 may store data, information, and the like generated while the analysis apparatus 600 analyzes the T cell activation.

The interface device 640 is a device that receives predetermined commands and data from the outside. The interface device 640 may receive genetic data of a patient from a physically connected input device or an external storage device.

Alternatively, the interface device 640 may receive an amino acid sequence of MHC and/or an amino acid sequence of antigen to be analyzed.

The interface device 640 may receive a learning model for data analysis. The interface device 640 may receive training data, information, and parameter values for training a learning model.

The interface device 640 may receive a distance or proximity frequency of a specific amino acid pair in a protein structure from a protein structure DB.

The communication device 650 means a configuration for receiving and transmitting predetermined information through a wired or wireless network. The communication device 650 may receive genetic data from an external object. The communication device 650 may also receive data for training a model. The communication device 650 may receive the amino acid sequence of MHC and/or the amino acid sequence of antigen to be analyzed.

The communication device 650 may transmit an analysis result of the input sample to an external object. The analysis result may be T cell activation for specific peptide-MHC. Alternatively, the analysis result may be whether the corresponding peptide is a neoantigen candidate in specific peptide-MHC.

The communication device 650 may receive a distance or proximity frequency of a specific amino acid pair in the protein structure from the protein structure DB.

The communication device 650 or the interface device 640 is a device that receives predetermined data or commands from the outside. The communication device 650 or the interface device 640 may be referred to as an input device.

The output device 660 is a device that outputs predetermined information. The output device 660 may output an interface necessary for a data processing process, an analysis result, and the like.

The arithmetic device 630 may identify the first amino acid sequence of MHC and the second amino acid sequence of antigen generated by tumor cells from genetic data. The arithmetic device 630 may identify the first amino acid sequence and/or the second amino acid sequence from genetic data using a specific program.

As described above, the arithmetic device 630 may produce a matrix for the first amino acid sequence and the second amino acid sequence by referring to the known protein structural information. The arithmetic device 630 may calculate the distance or proximity frequency of a specific amino acid pair to be evaluated by referring to the previously known protein structures from the protein structure DB. The arithmetic device 630 may determine an interaction value based on a distance or proximity frequency of a specific amino acid pair.

The arithmetic device 630 may predict whether T cell activation for specific peptide-MHC is present by inputting the interaction matrix to the neural network model. The arithmetic device 630 may predict the IFNγ secretion amount of T cell of specific peptide-MHC or whether the IFNγ is secreted. In addition, the arithmetic device 630 may determine the corresponding peptide as a neoantigen candidate when the T cell activation for specific peptide-MHC is high.

The arithmetic device 630 may be a device such as a processor, an AP, or a chip embedded with a program that processes data and processes a predetermined operation.

Hereinafter, experimental results verifying the effect of the above-described T cell activation method will be described.

Researchers performed ELISPOT analysis on EMT6 to verify the neural network model. Researchers selected a mutation with a variant allele frequency (VAF) greater than 0.3 from among the genes in the sample. Researchers selected 25 peptides (neoantigen candidates) with the highest score and 5 peptides (control group) with the lowest score based on the predicted score of the neural network.

Researchers performed ELISPOT analysis on each of the 25 and 5 peptides. Researchers performed ELISPOT analysis on H2-Dd/H2-Ld (class1 alleles) and H2-IAd, H2-IEd (class2 alleles). Researchers calculated ELISPOT analysis results (ELISPOT.count) for all 30 peptides. In addition, an in silico model for measuring the binding degree of peptide-MHC was used as a reference model. NetMHCIIpan was used as a reference model.

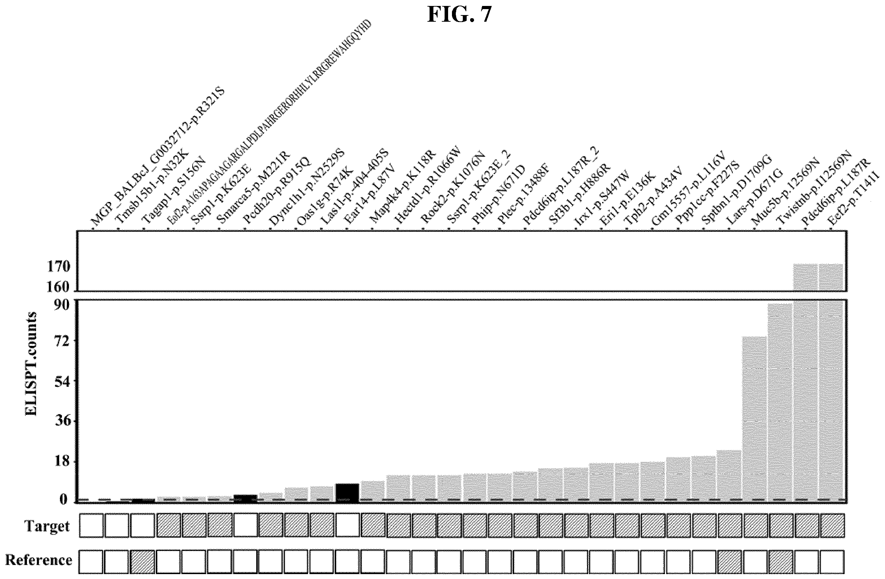

FIG. 7 is an example of experimental results verifying a neural network model. FIG. 7 illustrates ELISPOT analysis results for 30 peptides described above. FIG. 7 illustrates that peptides are arranged in the order of increasing ELISPOT.count value. That is, peptides with higher T cell activation are located on the right side of the graph of FIG. 7. The lower part of FIG. 7 illustrates prediction results of the above-described neural network model (marked as target) and a reference model (marked as Reference). A white block represents nonimmunogenic peptides, and a shaded block represents immunogenic peptides. Looking at the results of FIG. 7, it can be seen that the reference model used in many conventional studies does not properly predict the actual T cell activation. In contrast, the above-described neural network model showed overall high accuracy except for two peptides in the control group (nonimmunogenic). Therefore, it can be seen that the neural network model developed by researchers showed very superior performance compared to the conventionally widely used in silico model.

The data previously collected by the researchers are described in Table 1. Researchers selected some of the collected data as training data and trained models individually for the MHC I and the MHC II. In addition, researchers used some of the data as verification data. Researchers selected 13,128 for the MHC I and 6,650 for the MHC II. Researchers divided training data and verification data at a ratio of 7:3 for the selected data.

The accuracy of the neural network model was verified by comparing the result calculated by the neural network trained on the human or mouse peptide-MHC pair with the experimentally known value.

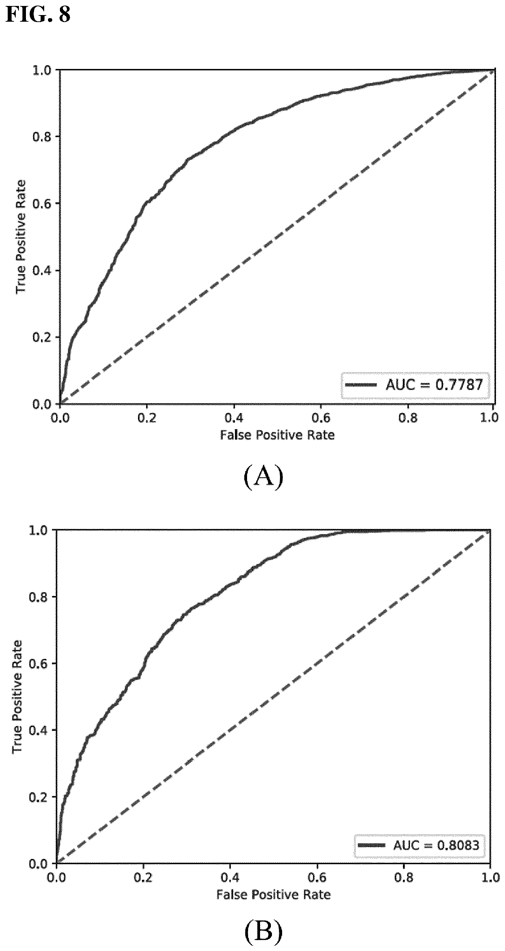

FIG. 8 is another example of experimental results verifying a neural network model. The neural network model trained individual models for MHC I and MHC II respectively. FIG. 8A is an experimental result for MHC I. As a result of the verification, an area under the curve (AUC) of the neural network model of MHC I was 0.7787. FIG. 8B is an experimental result for MHC II. The neural network model of MHC II had an AUC of 0.8083. Therefore, it can be said that the prediction accuracy of the developed neural network model is quite high.

In addition, the method of predicting T cell activation or the method of discovering a neoantigen discovery method as described above may be implemented as a program (or application) including an executable algorithm that may be executed on a computer. The program may be stored and provided in a non-transitory computer readable medium.

The non-transitory computer-readable medium is not a medium that stores data therein for a while, such as a register, a cache, and a memory, but means a medium that semi-permanently stores data therein and is readable by an apparatus. Specifically, various applications or programs described above may be provided by being stored in non-transitory readable media such as a compact disk (CD), a digital video disk (DVD), a hard disk, a Blu-ray disk, a USB, a memory card, a read-only memory (ROM), a programmable read only memory (PROM), an erasable PROM (EPROM), an electrically EPROM (EEPROM), or a flash memory.

The transitory readable media refer to various RAMs such as a static RAM (SRAM), a dynamic RAM (DRAM), a synchronous DRAM (SDRAM), a double data rate SDRAM (DDR SDRAM), an enhanced SDRAM (ESDRAM), a synclink DRAM (SLDRAM), and a direct rambus RAM (DRRAM).

The present embodiment and the drawings attached to the present specification only clearly show some of the technical ideas included in the above-described technology, and therefore, it will be apparent that all modifications and specific embodiments that can be easily inferred by those skilled in the art within the scope of the technical spirit included in the specification and drawings of the above-described technology are included in the scope of the above-described technology.

**Claims**

1. A method of predicting the T cell activation for peptide-major histocompatibility complex (MHC), comprising:

   receiving, by an analysis apparatus, genetic data of a patient;
   identifying, by the analysis apparatus, a first amino acid sequence of MHC and a second amino acid sequence of antigen generated by a tumor cell on the basis of the genetic data;
   producing, by the analysis apparatus, a matrix indicating an interrelationship between the first amino acid sequence and the second amino acid sequence in a single amino acid unit; and
   inputting, by the analysis apparatus, the matrix to a trained neural network model to determine whether the T cell secretes cytokine greater than or equal to a threshold value according to a binding of the MHC and the antigen.

2. The method of claim 1, wherein the matrix includes a degree of proximity of amino acid pairs in an actual protein structure based on previously known structural information of proteins for each of the amino acid pairs between the first amino acid sequence and the second amino acid sequence.

3. The method of claim 1, wherein the neural network model is trained using training data in advance, and
   the training data includes amino acid sequence pairs of MHC-neoantigen as input values and a cytokine secretion amount of T cells for each of the pairs as label values.

4. The method of claim 1, wherein the cytokine is interferon-γ.

5. The method of claim 1, wherein the analysis apparatus determines the antigen to be a target candidate for an anticancer vaccine when an output result of the neural network model is the cytokine secretion greater than or equal to the threshold value.

6. The method of claim 1, wherein the neural network model is a convolutional neural network (CNN), and the CNN outputs a degree of cytokine secretion of T cells for an input pair of the MHC and the antigen.

7. An analysis apparatus for predicting T cell activation for peptide-major histocompatibility complex (MHC), comprising:

   an input device configured to receive genetic data of a patient;
   a storage device configured to store a neural network model that predicts a cytokine secretion amount of a T cell based on a matrix representing an interrelationship of an amino acid sequence of MHC and an amino acid sequence of antigen generated by a tumor cell; and
   an arithmetic device configured to identify a first amino acid sequence of the MHC and a second amino acid sequence of the antigen generated by the tumor cell from the genetic data, produce a matrix representing the interrelationship between the first amino acid sequence and the second amino acid sequence in a single amino acid unit, and input the produced matrix to the neural network model to determine whether the MHC-antigen of the patient induces interferon-γ secretion of the T cell.

8. The analysis apparatus of claim 7, wherein the matrix includes a degree of proximity of amino acid pairs in an actual protein structure based on previously known structural information of proteins for each of the amino acid pairs between the first amino acid sequence and the second amino acid sequence.

9. The analysis apparatus of claim 7, wherein the neural network model is trained using training data in advance, and the training data includes amino acid sequence pairs of MHC-neoantigen as input values and an interferon-γ secretion amount of T cells for each of the pairs as label values.

10. The analysis apparatus of claim 7, wherein the analysis apparatus determines the antigen to be a target candidate for an anticancer vaccine when an output result of the neural network model is the interferon-γ secretion greater than or equal to the threshold value.

11. The analysis apparatus of claim 7, wherein the neural network model is a convolutional neural network (CNN), and the CNN outputs a degree of interferon-γ secretion of T cells for an input pair of the MHC and the antigen.

## FIG. 1

ANALYZE GENE OF SAMPLE (PATIENT)

**100**

DB

GENETIC DATA

PREDICT T CELL ACTIVATION OF pMHC

ANALYSIS RESULT

GENETIC DATA

PREDICT T CELL ACTIVATION OF pMHC

ANALYSIS RESULT

GENETIC DATA

PREDICT T CELL ACTIVATION OF pMHC

ANALYSIS RESULT

## FIG. 2

**200**

START

CONSTRUCT NEURAL NETWORK MODEL IN ADVANCE — 210

IDENTIFY ONE OR MORE TUMOR-SPECIFIC ANTIGENS BY ANALYZING GENE SEQUENCE FOR SAMPLE OF PATIENT — 220

PREDICT T CELL ACTIVATION FOR PEPTIDE-MHC USING NEURAL NETWORK MODEL FOR SPECIFIC ANTIGEN — 230

SELECT NEXT SPECIFIC ANTIGEN — 270

T CELL ACTIVATION ≥ THRESHOLD VALUE? — 240

NO

YES

ADD CURRENT PEPTIDE TO TARGET CANDIDATE GROUP — 250

IS PREDICTION OF T CELL ACTIVATION FOR SPECIFIC ANTIGEN ENDED? — 260

NO

YES

EXPERIMENT VERIFICATION FOR TARGET CANDIDATE GROUP (IF NECESSARY) — 280

DESIGN ANTICANCER VACCINE USING TARGET ANTIGEN — 290

END

## FIG. 3

| No. | MHC | Peptide | Cytokine(IFN-γ) |
|---|---|---|---|
| 1 | class I | AAGIGILTV | H |
| 2 | class I | ALWGFVPVL | L |
| ⋮ | ⋮ | ⋮ | ⋮ |
| n | class II | GILEFVFTL | H |

# FIG. 4

A

EXTRACT DEGREE
OF PROXIMITY OF
AMINO ACID PAIR
(420)

B

INPUT GENE
SEQUENCE OR
AMINO ACID
SEQUENCE (410)

PROTEIN
STRUCTURE
DB

PRODUCE MATRIX OF AMINO ACID
PAIR(430)

400

INTERACTION MATRIX

FIG. 5

ANALYSIS APPARATUS

500

ATGACGGAATATAAGCTGGTGGTG
ATCCAGCTGATCCAGAACCATTTT
CGGAAGCAGGTGGTCATTGATGGG
CAGGAGGAGTACAGCGCCATGCGG
GTGTTTGCCATCAACAACACCAAG

510

520

530

T CELL
ACTIVATION
OR
INACTIVATION

540

# FIG. 6

ANALYSIS APPARATUS

**600**

| | |
|---|---|
| STORAGE DEVICE (610) | INTERFACE DEVICE (640) |
| MEMORY (620) | COMMUNICATION DEVICE (650) |
| ARITHMETIC DEVICE (630) | OUTPUT DEVICE (660) |

**FIG. 7**

**FIG. 8**

(A)

(B)

## INTERNATIONAL SEARCH REPORT

| International application No. |
| --- |
| **PCT/KR2021/019182** |

| A. | CLASSIFICATION OF SUBJECT MATTER |
| --- | --- |

**G16B 5/00**(2019.01)i; **G16B 30/10**(2019.01)i; **G16B 40/20**(2019.01)i; **G16B 20/20**(2019.01)i; **G06N 3/08**(2006.01)i;
**G06N 3/04**(2006.01)i; **G06N 20/00**(2019.01)i; **C12Q 1/6869**(2018.01)i

According to International Patent Classification (IPC) or to both national classification and IPC

| B. | FIELDS SEARCHED |
| --- | --- |

Minimum documentation searched (classification system followed by classification symbols)

G16B 5/00(2019.01); C12N 13/00(2006.01); G01N 33/53(2006.01); G01N 33/68(2006.01); G06F 19/18(2011.01);
G06F 19/22(2011.01); G06F 19/24(2011.01); G06F 19/26(2011.01); G16B 20/30(2019.01); G16B 30/10(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 펩타이드-MHC(peptide-MHC), 신생항원(neoantigen), 아미노산 서열(amino acid sequence), 매트릭스(matrix), 신경망 모델(neural network model), 사이토카인(cytokine), 인터페론 감마(interferon-gamma)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | KR 10-2184720 B1 (KOREA ADVANCED INSTITUTE OF SCIENCE AND TECHNOLOGY) 30 November 2020 (2020-11-30) See claims 1, 6 and 12; and paragraphs [0077], [0078] and [0087]. | 1-11 |
| Y | WO 2020-180648 A1 (GRITSTONE ONCOLOGY, INC.) 10 September 2020 (2020-09-10) See claims 138 and 139; and paragraph [0030]. | 1-11 |
| Y | KIM, K. et al. Predicting clinical benefit of immunotherapy by antigenic or functional mutations affecting tumour immunogenicity. Nature Communications. 2020, vol. 11, article no. 951, pp. 1-11. See abstract; and pages 2-4 and 9. | 1-11 |
| A | KR 10-2017-0078619 A (THE JOHNS HOPKINS UNIVERSITY) 07 July 2017 (2017-07-07) See entire document. | 1-11 |
| A | KR 10-2016-0030101 A (BIONTECH AG et al.) 16 March 2016 (2016-03-16) See entire document. | 1-11 |

☑ Further documents are listed in the continuation of Box C.  ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **06 April 2022** | **08 April 2022** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.

**PCT/KR2021/019182**

**C.     DOCUMENTS CONSIDERED TO BE RELEVANT**

| Category* | Citation of document, with indication, where appropriate, of the relevant passages | Relevant to claim No. |
|---|---|---|
| A | KR 10-2018-0052959 A (KOREA INSTITUTE OF SCIENCE & TECHNOLOGY INFORMATION) 21 May 2018 (2018-05-21)<br>    See entire document. | 1-11 |

Form PCT/ISA/210 (second sheet) (July 2019)

**INTERNATIONAL SEARCH REPORT**
**Information on patent family members**

International application No.

**PCT/KR2021/019182**

| Patent document cited in search report | | | Publication date (day/month/year) | Patent family member(s) | | | Publication date (day/month/year) |
|---|---|---|---|---|---|---|---|
| KR | 10-2184720 | B1 | 30 November 2020 | WO | 2021-071182 | A1 | 15 April 2021 |
| WO | 2020-180648 | A1 | 10 September 2020 | AU | 2020-232211 | A1 | 30 September 2021 |
| | | | | CA | 3132072 | A1 | 10 September 2020 |
| | | | | EP | 3931350 | A1 | 05 January 2022 |
| | | | | IL | 285946 | A | 31 October 2021 |
| | | | | KR | 10-2022-0019655 | A | 17 February 2022 |
| KR | 10-2017-0078619 | A | 07 July 2017 | AU | 2015-317712 | A1 | 27 April 2017 |
| | | | | AU | 2015-317712 | B2 | 17 January 2019 |
| | | | | AU | 2019-202508 | A1 | 02 May 2019 |
| | | | | AU | 2019-202508 | B2 | 01 July 2021 |
| | | | | CA | 2961749 | A1 | 24 March 2016 |
| | | | | CA | 2961749 | C | 12 January 2021 |
| | | | | CA | 3017170 | A1 | 24 March 2016 |
| | | | | CA | 3017170 | C | 23 March 2021 |
| | | | | CN | 107002038 | A | 01 August 2017 |
| | | | | CN | 107002038 | B | 15 October 2021 |
| | | | | CN | 113791208 | A | 14 December 2021 |
| | | | | EP | 3194577 | A1 | 26 July 2017 |
| | | | | IL | 251261 | A | 29 May 2017 |
| | | | | IL | 251261 | B | 31 August 2020 |
| | | | | JP | 2017-529080 | A | 05 October 2017 |
| | | | | JP | 2021-006051 | A | 21 January 2021 |
| | | | | KR | 10-2020-0055808 | A | 21 May 2020 |
| | | | | KR | 10-2021-0032011 | A | 23 March 2021 |
| | | | | KR | 10-2229421 | B1 | 19 March 2021 |
| | | | | MX | 2017003625 | A | 11 October 2017 |
| | | | | SG | 11201702191 | A | 27 April 2017 |
| | | | | US | 10098939 | B2 | 16 October 2018 |
| | | | | US | 10987412 | B2 | 27 April 2021 |
| | | | | US | 2017-0246277 | A1 | 31 August 2017 |
| | | | | US | 2018-0043003 | A1 | 15 February 2018 |
| | | | | US | 2021-0252121 | A1 | 19 August 2021 |
| | | | | WO | 2016-044530 | A1 | 24 March 2016 |
| KR | 10-2016-0030101 | A | 16 March 2016 | AU | 2014-264943 | A1 | 26 November 2015 |
| | | | | AU | 2014-264943 | B2 | 12 September 2019 |
| | | | | AU | 2019-275637 | A1 | 02 January 2020 |
| | | | | CA | 2911945 | A1 | 13 November 2014 |
| | | | | CN | 105451759 | A | 30 March 2016 |
| | | | | CN | 105451759 | B | 05 February 2021 |
| | | | | CN | 113219179 | A | 06 August 2021 |
| | | | | DK | 2994159 | T3 | 25 May 2021 |
| | | | | EP | 2994159 | A1 | 16 March 2016 |
| | | | | EP | 2994159 | B1 | 10 March 2021 |
| | | | | EP | 3895727 | A1 | 20 October 2021 |
| | | | | ES | 2871384 | T3 | 28 October 2021 |
| | | | | HK | 1215169 | A1 | 19 August 2016 |
| | | | | HR | P20210773 | T1 | 17 September 2021 |
| | | | | HU | E055146 | T2 | 29 November 2021 |
| | | | | IL | 242281 | A | 30 April 2020 |
| | | | | JP | 2016-521128 | A | 21 July 2016 |

Form PCT/ISA/210 (patent family annex) (July 2019)

**INTERNATIONAL SEARCH REPORT**
Information on patent family members

International application No.

**PCT/KR2021/019182**

| Patent document cited in search report | | | Publication date (day/month/year) | Patent family member(s) | | | Publication date (day/month/year) |
|---|---|---|---|---|---|---|---|
| | | | | JP | 2020-103297 | A | 09 July 2020 |
| | | | | JP | 2021-121801 | A | 26 August 2021 |
| | | | | JP | 6710634 | B2 | 17 June 2020 |
| | | | | JP | 6882550 | B2 | 02 June 2021 |
| | | | | MX | 2015015511 | A | 09 December 2016 |
| | | | | NZ | 714059 | A | 27 August 2021 |
| | | | | SG | 11201508816 | A | 27 November 2015 |
| | | | | US | 1222711 | B2 | 11 January 2022 |
| | | | | US | 2016-0125129 | A1 | 05 May 2016 |
| | | | | WO | 2014-180490 | A1 | 13 November 2014 |
| | | | | WO | 2014-180569 | A1 | 13 November 2014 |
| | | | | ZA | 201508048 | B | 31 May 2017 |
| KR | 10-2018-0052959 | A | 21 May 2018 | KR | 10-1925040 | B1 | 04 December 2018 |

Form PCT/ISA/210 (patent family annex) (July 2019)